# EUROPEAN PATENT SPECIFICATION

(11) **EP 1 402 915 B1**
(45) Date of publication and mention of the grant of the patent: **07.12.2005**
(21) Application number: 03021831.7
(22) Date of filing: 26.09.2003
(51) Int. Cl.: A61M 16/06, A61M 16/12, A61M 16/20

(54) **Gas delivery mask with expiratory-, inspiratory- and dilution valves**
Beatmungsmaske mit Exspirations-, Inspirations- und Verdünnungsventilen
Masque destiné à l'inhalation d'un gaz avec des vannes d'inspiration, d'expiration et de dilution

(30) Priority: 27.09.2002 US 259997; 12.05.2003 US 437409
(43) Date of publication of application: 31.03.2004
(73) Proprietor: Viasys Healthcare GmbH, 97204 Höchberg (DE)
(72) Inventor: Stenzler, Alex, Yorba Linda, California 92887-4645 (US); Quinn, Tim, Yorba Linda, California 92887-4645 (US); Chu, Edmond, Yorba Linda, California 92887-4645 (US); Fisher, Joseph, Toronto General Hospital, Toronto, Ontario M5G 2C4 (CA); Iscoe, Steve, Kingston, Ontario K7L4T3 (CA); Preiss, David, Thornhill, Ontario L4J4K6 (CA); Prisman, Eitan, Thornhill, Ontario L4J3H5 (CA); Sasano, Hiroshi, Nagoyo 466-6084 (JP); Somogyi, Ron B., Toronto, Ontario M2R2S6 (CA); Vesely, Alex, Toronto, Ontario M5M2P7 (CA); Volgyesi, George, Toronto, Ontario M2J2P5 (CA)
(74) Representative: Heusler, Wolfgang

(56) References cited:
- GB-A- 659 225
- GB-A- 2 285 396
- US-A- 3 977 432
- US-A- 4 848 333
- US-A- 5 265 595
- US-A- 5 730 122
- US-A1- 2002 069 870

## Description

### Field of the Invention

The field of the present invention relates to a mask assembly for delivery of oxygen and other gases.

### Background of the Invention

Hypoxemia is a deficient oxygenation of the blood. The deficiency of oxygen may result from acute respiratory failure stemming from conditions such as pneumonia, heart disease, trauma to the chest or other etiologies. Conditions causing acute respiratory distress are also often associated with hyperventilation (high levels of breathing). Paradoxically, the patient's high minute ventilations and associated high inspiratory flows during hyperventilation severely limit the fraction of inspired oxygen (F_{I}O₂) that most oxygen (O₂) masks can deliver. When a sufficiently high arterial oxygen partial pressure (PaO₂) cannot be provided by mask alone, the therapeutic option defaults to endotracheal intubation. Since endotracheal intubation is associated with considerable discomfort, morbidity and cost, this step is not to be undertaken without exhausting other options.

The treatment objective for severe hypoxemia resulting from acute respiratory failure is to optimize the oxygen flowing to the alveoli and, thereby, increasing arterial PO₂. The effective delivery of high concentrations of O₂ depends on the capability of a mask to match the O₂ flow to the patient's minute ventilation and peak inspiratory flow without limiting the F_{I}O₂. One common approach for delivering oxygen using a mask is to try to match the patient's peak inspiratory flows with O₂ flowing to the mask. Peak inspiratory flows in breathless patients, however, can reach several hundred liters per minute; and most O₂ flowmeters are calibrated to only 15 L/min. Even when set at "flush," the upper limit of the flowmeter is still far less than peak flow requirements. Hence, delivery of higher O₂ flows into the mask in this situation requires a tandem set-up of multiple flowmeters, which increases the complexity and cost of the delivery system.

Another approach for oxygen delivery is to use a mask with an O₂ reservoir on the inspiratory side with or without a one-way valve between the reservoir and the mask. A mask with a valve between the reservoir and the mask is known as a non-rebreathing mask ("NRM"), while: a mask without the one-way valve between the reservoir and the mask is known as partial rebreathing mask ("PRM"). In theory, the reservoir fills with O₂ during exhalation and is available to meet peak inspiratory flow demands during inspiration. In practice, however, F_{I}O₂ is limited because there is an obligatory entrainment of room air throughout inspiration. Most conventional oxygen masks dilute the inspired oxygen with entrained room air because of the presence of ports on the mask through which the patient also exhales. Oxygen is also diluted because of poor fit of the mask to the face. In this case, the gas filling the alveoli and airways is the average diluted concentration, rather than the concentration of the supplied oxygen. Hence, during inspiration, these entrainment pathways provide a large source of dilution of the oxygen and reduce the F_{I}O₂.

Furthermore, the volume of entrained air depends on the relative resistance to flow in the portholes of the mask and the O₂ inlet. The difference in performance between the NRM and PRM may be small in this situation. On the one hand, the valve at the O₂ inlet prevents expired gas with lower PO₂ from entering the reservoir; on the other hand, it increases the resistance to flow from the bag to the mask and thus results in entrainment of more air that further decreases the F_{I}O₂. These considerations apply even when the mask may fit well on the face of the patient.

Another consideration for delivery of oxygen using a mask is the risk of oxygen flow failure because either the oxygen supply is exhausted or the oxygen flow path may be blocked. In this situation, an anti-asphyxiation valve allows inhalation of room air. For example, a mask called the BLB mask was developed around the time of World War II for pilots who required supplemental oxygen while flying at higher altitude. The two-part form of claim 1 is based on the technical features of a BLB mash. It used an external oxygen source that filled an inspiratory reservoir. The pilots inhaled through a one-way valve between the mask and the reservoir. They exhaled through a second valve in the mask. The operation of the BLB mask called for supplying an oxygen flow sufficient to meet the pilot's ventilatory requirements. If the pilot required more oxygen than was present in the reservoir, the BLB mask contained an anti-asphyxiation valve to enable the pilot to inhale room air rather than asphyxiate in these situations. The anti-asphyxiation valve is acceptable only for emergency situations and for short periods of time. For extended periods of time, breathing through the anti-asphyxiation valve leads to fatigue. In the case of distress patients, this may not acceptable.

Another concern that may be associated with patients who are receiving oxygen is the air that they exhale. Many of these patients have respiratory infections and the pathogens in their exhaled breath such as viruses or bacteria can contaminate the environment around them, potentially exposing other patients or healthcare workers to these organisms. Some of these patients may be receiving inhaled medications while receiving oxygen, and the inhaled medications may be toxic or harmful to others if exhaled into the environment. Thus, there exists a need for an improved mask for efficient and safe delivery of oxygen or other gases to a patient or subject.

### Summary of the Invention

The present invention provides for a method and a device to deliver oxygen or other gases to a patient as defined in claim 1. This is possible at moderate oxygen flow rates. According to one aspect of the invention, a mask assembly is provided for delivering oxygen or other gases to a patient by sequentially diluting room air to the oxygen flow during a respiratory cycle of the patient wherein the room air is inspired at the end of inspiration. In particular, high concentration of oxygen or other gases from the gas source is first delivered to the alveoli of the lung before substantial volume of room air is allowed to enter the oxygen or gas flow path and dilute the flow of oxygen or gas. Since the space between the nose and the alveoli (anatomic dead space) does not participate in gas exchange, the sequential inhalation of, for example, oxygen then room air results in the alveoli receiving high concentration of O₂ while room air inspired at end inspiration is delivered to the anatomical deadspace. Thus, by sequentially adding room air to be inspired at the end of inspiration, less oxygen is used in total and lower flow rates can be utilized.

The device is a mask assembly that comprises a gas reservoir and a housing attached to the reservoir bag. The housing comprises a gas intake port (adapted to connect to a gas source) and a valve system that controls the flow of gases such that the gas flowing to the subject is sequentially diluted with room air during a respiratory cycle without inducing fatigue. In another embodiment, the mask assembly may also be comprised of inspiratory and expiratory flow paths each, preferably containing a low resistance one-way valve. The mask further comprises a sequential dilution valve that controls the flow of air from the atmosphere to the inspiratory flow path. The dilution valve is also a low resistance valve but has a cracking pressure and resistance slightly greater than the one-way valve of the inspiratory flow path. A gas reservoir such as a reservoir bag may be attached to the inspiratory flow path and may be filled with oxygen or other gases during expiration. During inspiration, oxygen or other gases are preferentially drawn from the gas source and the gas reservoir. When all of the gas from the gas reservoir is depleted, the dilution valve opens to supply additional room air from the atmosphere to meet the patient's tidal volume.

In another embodiment of the invention, the gas mask further comprises a flexible face piece that conforms to the face of the patient. The face piece does not contain any portholes and is attached to a housing comprising the inspiratory and expiratory flow paths and the valves. The face piece may also include straps for securing the face piece tightly on the face of the patient.

In another aspect of the invention, a filter may be positioned along the expiratory flow path such that infectious agents, toxic chemicals, or other harmful agents may be removed from the expiratory flow before exiting to the atmosphere. Preferably, the filter may be positioned downstream of a one-way valve that opens in the direction of the expiratory flow. In one embodiment, the filter may be of sufficient porosity to capture bacteria, viruses, or aerosol particles carrying these infectious agents, thereby minimizing exposure of other patients or healthcare workers to these organisms. In another embodiment, excess medications such as anesthetic gas may be removed by the filter from the expired air to minimize exposure of others. In yet another embodiment, carbon monoxide may also be filtered from the expired air before it exits to the environment while oxygen is being delivered to the patient suffering from carbon monoxide poisoning.

These and other features and advantages of the preferred embodiment will be described below in conjunction with the figures.

### Brief Description of the Drawings

FIG. 1 depicts a mask assembly according to one embodiment of the present invention.
FIG. 2 depicts the flow of gas during inspiration according to one embodiment of the present invention.
FIG. 3 depicts the flow of gas during expiration according to one embodiment of the present invention.
FIG. 4 depicts the comparison of resistances between the inspiratory and dilution valves according to one embodiment of the present invention and the inspiratory and anti-asphyxiation valve of a prior BLB mask.
FIG. 5 depicts a representation of the anatomical deadspace of the respiratory system.
FIG. 6 depicts the results of measuring the fraction of oxygen inspired (F_{I}O₂) in a normal breathing subject using a mask assembly according to one embodiment of the present invention.
FIG. 7 depicts a mask assembly including a filter connected to the expiratory flow path.

### Detailed Description of the Preferred Embodiments

Although the embodiments of the invention are described in conjunction with the delivery of oxygen to patients, these embodiments can also be applied to use in delivery of other gases such as helium-oxygen mixtures, nitric oxide, gas anesthetics, and any other gases used for inhalation and to other subjects aside from medical patients. The use of oxygen in this description is not meant to limit the application of the described method and devices to oxygen.

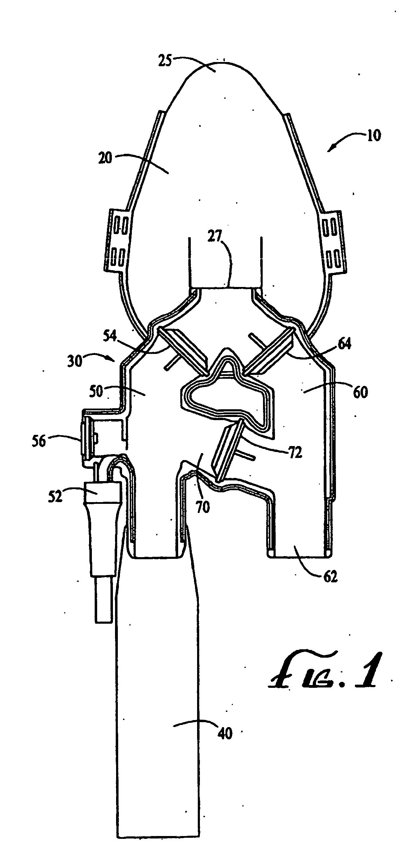

Figure 1 shows a mask assembly 10 according to one embodiment of the present invention. The mask assembly 10 generally comprises a face piece 20, a hollow manifold housing 30, and a gas reservoir bag 40. A lumen 27 is provided on the face piece 20 that is adapted to receive the manifold housing 30 such that the face piece 20 and the manifold housing 30 are in fluid connection with each other. Likewise, the gas reservoir bag 40 is attached to the manifold housing 30 such that they are in fluid connection with the each other. The gas reservoir bag 40, preferably having a capacity of 0.5 to 1 liter, may be made out of any collapsible material.

With respect to the face piece 20, it can be configured to fit over the patient's nose or mouth, or both, and can be made out of plastic, vinyl, silicone, or any other suitable materials. In a preferred embodiment, the face piece 20 covers the patient's nose and mouth and is flexible with a preferred durometer range of 60 to 90 to allow for the face piece 20 to form to the face of the patient (although other durometers may also be used). Elastic straps attached to the side of the face piece 20 can be used to secure the face piece 20 to the patient's face. Additionally, to prevent the mask from sliding up the patient's forehead, the tip 25 of the face piece 20 may be trimmed to sit at the bridge of the patient's nose, and a foam strip may be placed inside of the bridge portion of the face piece 20. A V-shaped metal strip may also be placed at the bridge section to hold the shape of the nose and provide better sealing.

The manifold housing 30 is generally a hollow structure and may be comprised of tubing that form an inspiratory limb or flow path 50 and an expiratory limb or flow path 60. The inspiratory flow path 50 directs the oxygen supplied through a gas intake port 52 to the face piece 20. The expiratory flow path 60 directs the exhaled gas from the face piece 20 out to the atmosphere through an exit port 62. The manifold housing 30 may further comprise a conduit 70 that feeds room air from the atmosphere into the inspiratory flow path 50. The reservoir bag 40 may be positioned and attached at the end of the tubing for the inspiratory flow path 50. The manifold housing 30 can be constructed of clear, rigid plastic such as polystyrene or polycarbonate by molding two halves of the plastic and securing them together by sonic welding. Alternatively, the manifold housing 30 may also be made or assembled from flexible tubing such as rubber, silicone, or any other suitable materials. Although described in certain preferred embodiments, the construction of the housing can be in any color, opacity, hardness, and materials.

Within the manifold housing 30 are preferably positioned three one-way valves that control the flow of gases. First, an inspiratory valve 54 may be positioned in the inspiratory flow path 50 between the face piece 20 and the reservoir bag 40. The inspiratory valve 54 opens in the direction of the inspiratory flow. Second, an expiratory valve 64 may be positioned in the expiratory flow path 60, and expiratory valve 64 opens in the direction of the expiratory flow that leads to the atmosphere. Finally; a dilution valve 72 may be placed in the conduit 70, and the dilution valve 72 opens in the direction that controls the entry of room air into the inspiratory flow path 50. Although the valves, as shown in FIG. 1 are placed inside the tubings of the manifold housing 30, the valves may alternatively be positioned along the walls of the tubings or the face piece 20. For example, in one embodiment, the dilution valve 72 may be positioned at a port along the wall of the inspiration flow path 50. In another embodiment, the dilution valve 72 may be positioned at a port on the face piece 20. In yet another embodiment, the mask may be of a kind similar to a partial rebreathing mask that does not include an inspiratory valve, since in breathing 100% oxygen, exhaled air can contain as much as 95-97% oxygen (i.e., only 3-5 percent of oxygen is consumed during respiration). Thus, the position and number of the valves may be varied but still achieve the objective of the present invention.

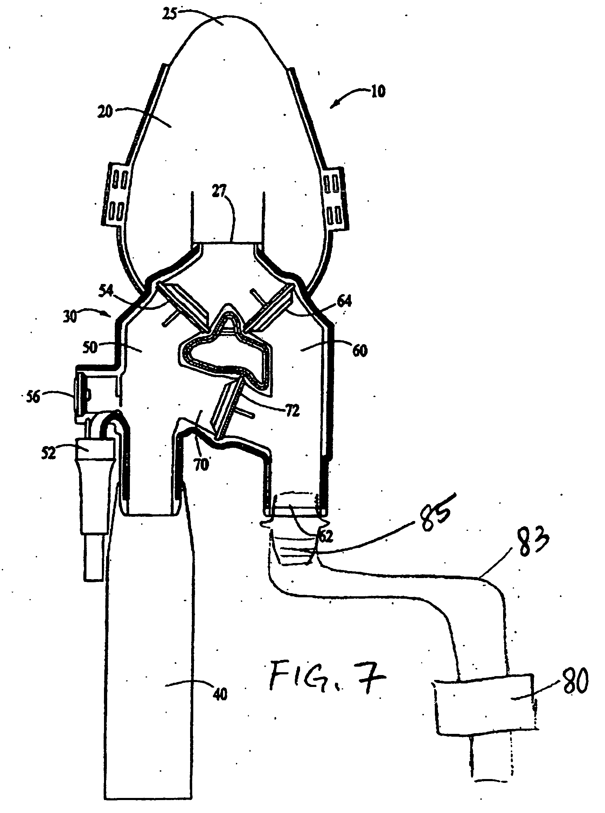

Additionally, the mask assembly may also include or be connected to a filter positioned along the expiratory flow path 60. It is contemplated that the filter can be positioned upstream or downstream of the expiratory valve 64. It may be preferred, but not required, for the filter 80 to be positioned downstream from the expiratory valve. 64 (as shown in Figure 7) for ease of replacement, if necessary. The filter 80 is preferably detachably connected to the exit port 62 and may be connected to the exit port 62 using any suitable mating fitting 85 (such as a 22mm ID fitting). The filter 80 may be connected to the exit port 62 directly or through a tubing 83 as shown in Figure 7. Any type of filters may be used depending on the type of agents to be removed from the expired air before it exits to the atmosphere. For example, if a patient using the mask is suffering from a contagious infectious disease such as Severe Acute Respiratory Syndrome ("SARS"), tuberculosis, whooping cough, flu (influenza), or any other infection, it would be desirable to remove these infectious agents from the expired air before it exits to the atmosphere so as to minimize the transmission of the infectious agents to another. In this example, porous filter materials may be used to capture the aerosol particles or droplets from the expired air that may carry the infectious agents much like a surgeon masks used during surgical operation. If needed, filter materials with smaller porosity may also be used to capture bacteria or viruses. HEPA filters may also be used to capture even smaller viruses. Examples of commercially available gas filters suitable for this use include Allegiance Airlife™ (#001851) Bacteria/Viral Filter and Allegiance Airlife™ (#001852) HEPA Filter.

In another example, if a patient is receiving or has received anesthesia, it may be desirable to remove any anesthesia from the expired air before it exits to the atmosphere so as not to expose another person in the room to the anesthesia. Additionally, a patient may be undergoing recovery from a surgery in which anesthetic gas was used and wherein oxygen is administered to the patient to clear out excess anesthetic gas in the patient's system. The filter 80 may be a silica zeolite filter or any other type of scavenger that may be able to remove the anesthesia. An example of a commercially available scavenger for anesthesia is the Bluezone Isoflurane filter.

In yet another example, if a patient who has been exposed to carbon monoxide is receiving oxygen through the mask to clear the carbon monoxide from its blood, it may be desirable to remove any excess carbon monoxide from the expired air before it exits to the atmosphere. An example of a commercially available filter for stripping carbon monoxide gas is a Evac-u8™ Hopcalite Filter.

Although in the preferred embodiment, the mask assembly is described as having three one-way valves that control the flow of gases, it is also contemplated that various configurations of valves can also be used. For example, the mask assembly may be used with the filter 80 even though it does not have the dilution valve 72. A mask with only an inspiratory valve 54 and an expiratory valve 64 would still be able to direct all expired air to exit through the exit port 62 and the filter 80. Even a mask with no valves, or that includes only the dilution valve 72 or only the expiratory valve 64, and having only one exit port 62 can be used in conjunction with a filter 80 to remove undesired agents from the expired air, albeit some of these undesired agents may travel into and contaminate the inspiratory flow path 50, the reservoir bag 40, or the gas source. In this embodiment, it may be desirable to position another filter between the gas source and the gas intake port 52 to reduce contamination of the gas source. Since most masks are disposable, contamination of the inspiratory flow path 50 and reservoir bag 40 does not pose serious problems.

Regarding the valves of the preferred embodiment, these valves may be any type of one-way valves known in the art. Valves of different properties may be obtained from, commercial sources such as from Hans Rudolph, Inc. (Kansas City; MO, USA). In a preferred embodiment, each valve is composed of a plastic-molded seat and mushroom-shape flap leaf that is made out of silicone or rubber. On the one hand, when gas flow pushes the leaf against the seat, the valve prevents gas from going through the valve. On the other hand, flow is allowed to go through the valve when it comes from underneath the seat and lifts the flap leaf away from the seat. Each leaf may have a stem that extends through a port on the plastic seat and that is used to secure the flap leaf mechanically to the seat. Desired resistance of the valve may be achieved depending on the stiffness or durometer of the flap leaf selected and the size of the porthole through which the stem extends. In one embodiment, the valves are captured in place by sitting in the cavity formed by the two halves of the plastic manifold housing 30.

To minimize the effort required for breathing, the flow resistances of all inspiratory, expiratory, and dilution valves are preferably low. The dilution valve has a slightly higher resistance than the inspiratory and/or the expiratory valves. For example, the flow resistance of the dilution valve 72 is in the range of less than 4 cmH₂O/l/sec at a flow rate of 60 liters per minute, and even more preferred at a range less than 3 cmH₂O/l/sec, and most preferred at a range of less than 2 cmH₂O/l/sec at the same flow rate. The resistance of the inspiratory valve is also preferably lower than most leaks around the mask assembly. For example, the inspiratory valve is preferred to have a flow resistance of less than 2 cmH₂O/l/sec at a flow rate of 60 liters per minute, and more preferred to have a flow resistance of less than 1.5, and most preferred to have a resistance of less than 1.1 cmH₂O/l/sec at the same flow rate. Because the resistance of the dilution valve 72 is slightly greater than the inspiratory valve 54, sequential opening of the valves is achieved. Moreover, to allow for sequential dilution of room air, the cracking pressure of the dilution valve 72 is greater than the pressure needed to empty the reservoir bag 40.

An additional safety or anti-asphyxiation valve 56 may also be positioned on the inspiratory flow path 50 to allow for entry of room air in the case of emergency or oxygen source failure. Because the dilution valve 72 also allows for room air to enter the inspiratory flow path 50 when the oxygen flow is insufficient to meet the tidal volume of the patient, the safety or anti-asphyxiation valve 56 is redundant and may be provided for additional safety of the mask. In contrast to the anti-asphyxiation valve 56 and to a similar valve of the prior art BLB mask, the flow resistance of the dilution valve 72 according to the present invention is markedly lower than the resistance of the safety or anti-asphyxiation valve 56. The lower resistance of the dilution valve 72 allows the valve to be used as part of the normal breathing pattern of the patient or subject without fatigue to the patient. While higher resistances are acceptable for emergency situations and for short periods of time as used in the BLB mask, they are not safe for extended periods of time because it leads to breathing fatigue.

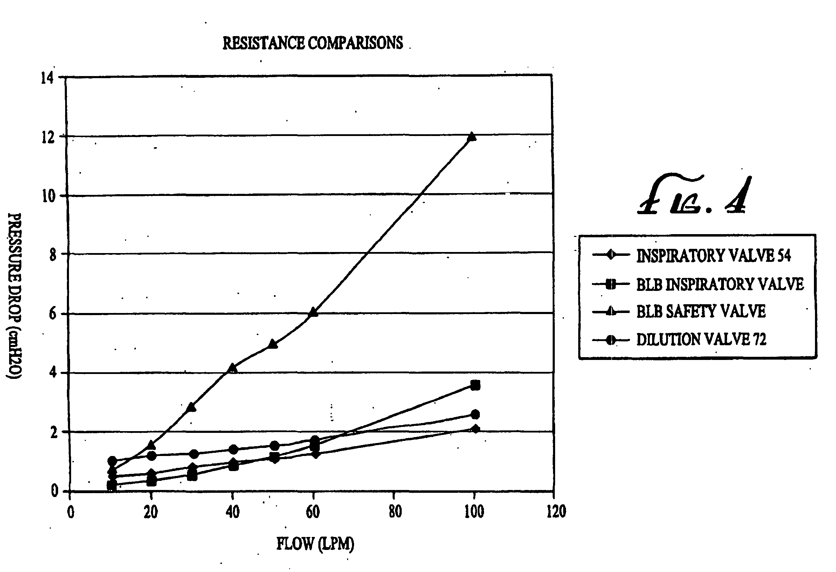

FIG. 4 shows the comparison of resistance between the BLB's inspiratory and anti-asphyxiation valve and the inspiratory valve 54 and dilution valve 72 according to one embodiment of the present invention. Resistances in flow rates of up to 100 liters were measured. As seen in FIG. 4, the inspiratory valve 54 and the dilution valve 72 according to one embodiment of the present invention perform at low resistance values of less than two cmH₂O/l/sec at a flow rate of 60 liters per minute. The normal inspiratory valve of the BLB mask also measured at a similarly low resistance. However, the resistance of the anti-asphyxiation valve of the BLB mask becomes excessive above 20 liters per minute with resistance values of greater than four cmH₂O/l/sec. Flow rates for quiet breathing in normal adults is typically around 30 liters per minute, and the United States Food and Drug Administration recommends that resistances for normal breathing should be below two cmH₂O/L/sec. Thus, the BLB's safety valve would add excessive workload to the subject breathing at rest and could not be used with moderate flow rates contemplated by the present invention.

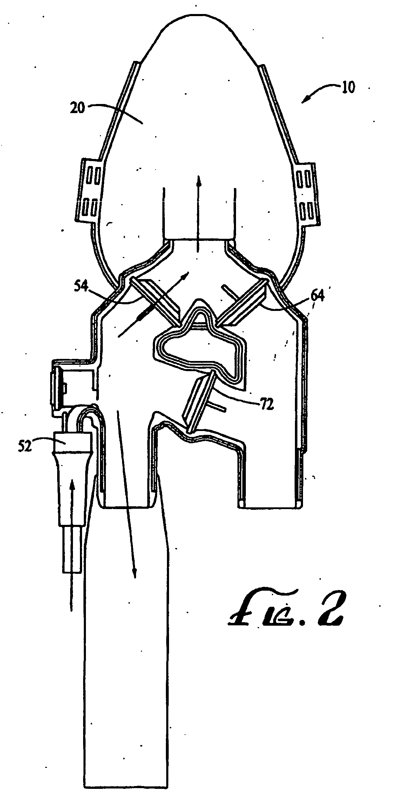

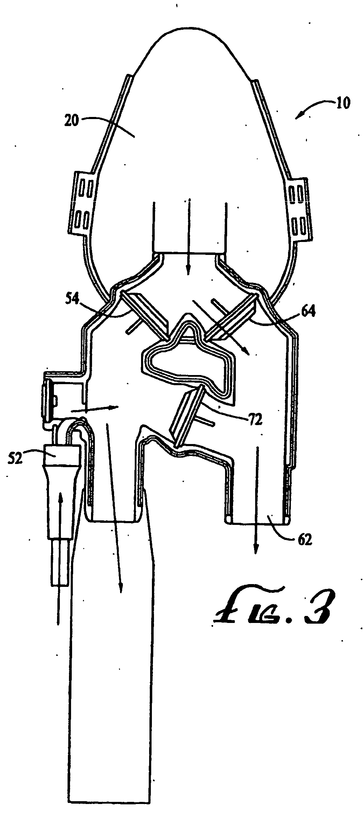

With reference to FIGS. 2 and 3, the use of the mask assembly 10 will now be described using the arrows in FIGS. 2 and 3 that show the flow of oxygen in the mask assembly 10. When the mask assembly 10 is connected to an oxygen supply (not shown), oxygen enters the mask assembly 10 through the gas intake port 52. During inspiration, negative pressure is created in the face piece 20 that lifts the flap leaf of the inspiratory valve 54 and allows oxygen to enter the face piece 20 to be inhaled by the patient (FIG. 2). As shown in FIG. 2, the oxygen source will also deliver and fill the gas reservoir bag 40 when the patient's demand does not exceed the supplied oxygen amount. The negative pressure on the face piece during inspiration also closes the expiratory valve 64 to prevent leakage of room air. During expiration as shown in FIG 3, the exhaled gas flow will push the leaf off the seat of the expiratory valve 64 allowing exhaled air to go through and exhaust to the atmosphere. At the same time, exhalation also pushes against the inspiratory valve 54 preventing the oxygen from going through valve and allowing oxygen to fill the gas reservoir bag 40.

If the total volume of oxygen flowing into the mask assembly 10 and the volume of oxygen in the gas reservoir bag 40 is equal to or greater than the minute ventilation of the patient, no atmospheric or room air is entrained and the patient gets pure oxygen. If, however, the minute ventilation or tidal volume of the patient exceeds the oxygen flowing into the mask assembly 10 and the oxygen stored in the gas reservoir bag 40, the reservoir bag 40 collapses. The dilution valve 72 subsequently opens, and the remainder of the inspired gas is drawn from the atmosphere. Because room air is not introduced into the inspiratory flow path 50 until after the reservoir bag 40 collapses, the flow of oxygen into the mask assembly can be adjusted such that the entrained room air fills only the anatomical deadspace of the respiratory system.

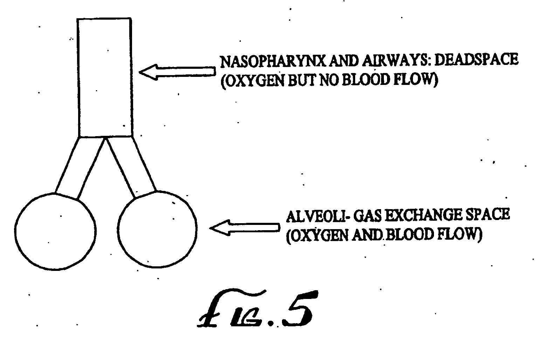

As depicted in FIG. 5, the space between the nose and alveoli is called deadspace because it does not participate in gas exchange. By sequentially diluting room air during inspiration in a respiratory cycle such that the room air inspired at the end of inspired fills the deadspace, the flow rates of the oxygen from the source can be decreased without reducing the efficiency of oxygen delivery to the alveoli. In normal circumstances, the flow rate of the oxygen into the mask assembly according to one embodiment of the invention may be in the range of at 1-15 liters per minute, more preferably in the range of 4-12 liters per minute, and most preferably in the range of 8-10 liters per minute. In contrast, normal flow rates using conventional masks often are in the range of 10-40 liters per minute. Although the range of flow rates for the conventional mask may overlap with the range for the mask assembly according to one embodiment of the present invention , the mask assembly according to one embodiment of the present invention requires lower flow rates to deliver equal volumes of oxygen to the alveoli when compared to a conventional mask.

The advantage of the mask assembly 10 according to one embodiment of the invention may be illustrated by the following mathematical example. Assume for a given patient that the following are true:
1. Tidal volume of the patient's breath is 600 ml.
2. The anatomical deadspace (or non-gas exchange volume) of the patient is 200 ml.
3. Respiratory rate of the patient is 12 breaths per minute, (i.e., respiratory cycle equals to 5 seconds).
4. The ratio of inspiration time to expiration time is 1:2, (i.e., for each respiratory cycle, it takes 1.67 seconds to inhale and 3.33 seconds to exhale).
5. Oxygen flowing into the mask is set at 5.5 liters per minute (1pm) or 92 ml/sec.,(i.e, 5500 ml/min / 60 sec = 92 ml/sec).
6. Mean inspiratory flow is 359 ml/sec (i.e. (600 ml / 1.67 (inspiratory time)).
7. All of the oxygen flowing is inhaled in each breath. Given the above assumptions, the volume of oxygen flowing into the mask assembly 10 during the respiratory cycle can be calculated to be 458 ml (i.e., (5,500ml/60 sec) x 5 sec 458ml). Since the anatomical deadspace is 200 ml, the alveolar volume is 600ml - 200ml = 400 ml. The volume of oxygen stored in the gas reservoir bag during exhalation is about 305 ml (i.e., (5,500 ml/60 sec) x 3.33 secs. = 305 ml).

In a conventional mask, oxygen is mixed with large volume of room air from the very beginning of inspiration. Thus, the average volume of oxygen inspired based on the patient's tidal volume is calculated from the oxygen flow volume during a respiratory cycle plus the difference between the tidal volume and oxygen flow volume multiplied by the percentage of oxygen in room air (i.e., (458 ml x 1.0) + ((600 ml - 458 ml) x .21) = 487 ml) . The average F_{I}O₂ is then 487/600 or 81%. Since the F_{I}O₂ is 81%, the volume of oxygen that actually reaches the patient's alveoli is 81% of the alveolar volume (i.e., 0.81 x 400 ml = 324 ml).

In contrast, the mask assembly 10 allows for all of the oxygen from the reservoir bag 40 to be inhaled before the sequential dilution valve 72 opens. Thus, the first gas into the alveoli is theoretically 100% oxygen. The total alveolar volume of oxygen can be calculated from the volume of oxygen in the reservoir bag plus the volume of oxygen flowing during inspiration. In this case, the 305 ml of oxygen from the reservoir bag enters the alveoli during the pre-dilution inspiration along with the 105 ml of oxygen flowing during the time to empty the reservoir bag (i.e., 359 ml/sec (patient inspiratory flow) - 92 ml/sec (supplied from oxygen flow) = 267 ml/sec from reservoir or 1.14 second to empty reservoir). This 410 ml completely fills the 400 ml of alveoli volume and no room air enters the alveoli. Since the total volume of the alveoli that is involved in gas exchange is assumed to be 400 ml, the equivalent F_{I}O₂ in the alveoli is 100% (410/400 = >100%). Therefore, due to the sequential delivery of oxygen and air, the minimum oxygen flow needed to provide an F_{I}O₂ of 1.0 is theoretically equal to the *alveolar*, and not the minute, ventilation, i.e., only about 2/3 to 3/4 of the minute ventilation at rest.

To match the F_{I}O₂ of 81% of the conventional gas masks under the same conditions where 324 ml of oxygen reaches the alveoli, the mask assembly 10 will- require only an oxygen flow of less than 4.2 liters per minute. The total volume of oxygen flowing from the gas source during a respiratory cycle is 350 ml (i.e., 4.2 liters per minute x 5 seconds). The volume of oxygen stored in the gas reservoir bag during exhalation is 233 ml (i.e., 4.2 liters per minute x 3.33 seconds). It will take 0.81 seconds to empty the reservoir bag (i.e., 359 ml/sec - 70 ml/sec =289 ml/sec (emptying flow rate from reservoir); 233ml / 289 ml/sec = 0.81 sec). Since substantial room air does not enter the mask until the reservoir bag is depleted, during the 0.81 seconds it takes to empty the reservoir, 290 ml of oxygen is first inspired (i.e., 233 ml + 57 ml (oxygen flow)) before another 110 ml of room air is inspired at the end of inspiration to fill the alveoli, (290 ml +110 ml = 400 ml alveolar volume). Since oxygen continues to flow during this period and room air is only 21% oxygen, an additional 22 ml of 100% oxygen and only 18 ml of the 88 ml room air is oxygen. In this case, the alveoli received 330 ml of oxygen (290 ml + 22 ml +18 ml = 330 ml), which is 83% of the alveolar volume. Based on this example, it can be seen that the mask assembly according to one embodiment of the invention is about 133% to 150% more efficient in delivering oxygen to the patient than conventional masks and can deliver less than 100% oxygen to patients at significantly lower oxygen flows.

When oxygen supply is low such as in emergency transport of injured patients or other emergencies, delivering less than 100% oxygen, or even less than 50% oxygen, may be preferred to prolong the supply of oxygen. In these situations, the mask assembly 10 may be greatly advantageous. For example, based on the above mathematical assumptions and using a flow rate of 1 liter per minute, F_{I}O₂ of 37.5% may be delivered to the alveoli. (Flow rate = 1,000 ml/60 sec = 16.7ml/sec; 16.7ml/sec x 3.33 sec = 55.6ml (reservoir volume); 16.7ml/sec x 1.67 sec = 27.9ml (oxygen flowing during inspiration); (55.6ml + 27.9ml)+(316.5ml x 0.21) = 150 ml (oxygen delivered to alveoli); 150ml /400ml = 37.5%). In contrast, conventional masks using a flow rate of 1 liter per minute delivers an F_{I}O₂ of 32%, which is significantly lower. (16.7ml/sec x 5 sec = 83.5 ml; (83.5 ml x 1) + ((600 ml - 83.5) x .21) = 192 ml; 192ml/600ml = 32%.)

As a further example, a mask assembly 10 as shown in FIGS.1-3, was constructed and tested, and it consistently delivered over 90% F_{I}O₂. Measurements were performed using a SensorMedics Vmax 229 metabolic measurement system (SensorMedics Corporation, Yorba Linda, California) in a breath-by-breath mode. The Vmax 229 system is an FDA approved device capable of measuring instantaneous flow, oxygen, and carbon dioxide in humans under a wide range of clinical ventilation levels. The system samples data in eight millisecond intervals and automatically aligns the signal to calculate oxygen uptake and other parameters. A sampling port was established through the sidewall of the mask's face piece that protruded into the mask and directly in front of the subject's nose and upper lip. The sampling line of the Vmax was attached to this sampling port. The flow sensor of the Vmax was attached with a 3-inch tube to the exhalation flow path of the mask assembly. The flow sensor was positioned to only record exhaled flow. Inspiration was assumed to begin when expiratory flow ceased.

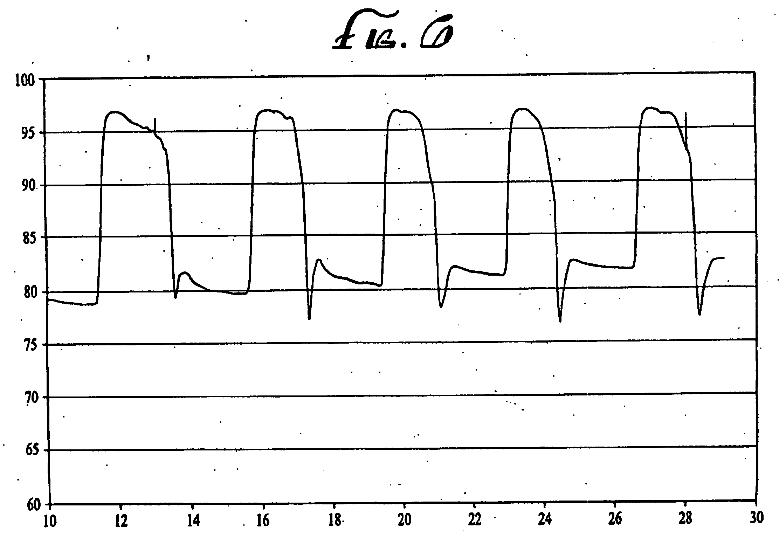

Flow to the mask was set at 8 liters per minute, and the mask assembly was placed on a normal volunteer who was instructed to breathe normally. Data was collected for a period of 10 minutes. As seen in FIG. 6, the inspired oxygen during normal breathing using the mask assembly embodiment of the present invention exceeded 90 percent in all breaths and in most breaths, exceeded 95%. Thus, at moderate flow rates such as 8 liters per minute, the mask assembly according to an embodiment of the present invention provides adequate flow for a normal subject without substantial dilution of the oxygen flow.

Some dilution with room air may occur at the beginning of the inspiration from small leaks around the mask assembly 10. This dilution, however, is minimal as seen in the above example where F_{I}O₂ greater than 95% is achieved. Substantial dilution does not occur until after the gas reservoir bag 40 is depleted and the dilution valve 72 opens. Hence, dilution with room air is sequentially achieved. In contrast, conventional masks allow substantially dilution of the oxygen flow with room air all through out the inspiration period and thus, dilution is not sequential.

While preferred embodiments of the present invention have been shown and described, various modifications may be made without departing from the scope of the present invention. For example, although the preferred embodiment employs a face piece 20 as shown in FIGS. 1-3, it is also contemplated as part of the invention that the valve system, as described above, may be used without a face piece 20. Tubings used for endotracheal intubation may similarly employ the valve system described above to control the flow of gas and to allow for sequential dilution of the oxygen with room air. Therefore, the invention should not be limited, except to the following claims.

## Claims

1. A mask assembly for delivering a gas for inhalation, the mask assembly comprising:
a face piece (20);
a gas reservoir (40) for holding the gas;
a housing (30) attached to the face piece (20) and reservoir (40), wherein the housing comprises:
a gas intake port (52) that feeds the gas to the face piece (20) and the gas reservoir (40);
a one-way expiratory valve (64) that opens in the direction of the expiratory flow leading to the atmosphere; and
a one-way inspiratory valve (54) positioned between the face piece (20) and the gas reservoir (40);
**characterized in that** the housing (30) further comprises
a dilution valve (72) that opens and allows room air to enter the housing (30), said dilution valve (72) having a cracking pressure greater than the pressure needed to empty the reservoir (40) during inspiration and a resistance equal or less than 4 cmH₂O/l/sec at a flow rate of 60 liters per minute.

2. The mask assembly according to claim 1 wherein the inspiratory valve (54) has a resistance equal or less than 2 cmH₂O/l/sec.

3. The mask assembly according to claim 1 wherein the expiratory valve (64) has a resistance equal or less than 2 cmH₂O/l/sec.

4. The mask assembly according to claim 1 wherein the inspiratory valve (54) has a resistance less than the dilution valve (72).

5. The mask assembly according to claim 1 further comprising an anti-asphyxiation valve (56).

6. The mask assembly according to claim 1 further comprising a strap attached to the face piece (20) for securing the mask assembly to a face of a subject.

7. The mask assembly according to claim 1 wherein the gas reservoir is a bag (40).

8. The mask assembly according to claim 1, wherein the housing (30) is.clear.

9. The mask assembly according to any preceding claim wherein the valves each comprise a valve seat having a port hole, a flap leaf attached to a stem, wherein the stem extends through the port hole to mechanically secure the flap on to the valve seat.

10. The mask assembly according to any preceding claim, wherein the housing comprises a filter (80) positioned along the expiratory flow.

11. The mask assembly according to claim 10 wherein the filter (80) is capable of filtering an infectious agent.

12. The mask assembly according to claim 10 wherein the filter (80) is capable of filtering an anesthetic gas.

13. The mask assembly according to claim 10 wherein the filter (80) is capable of filtering carbon monoxide.

14. The mask assembly according to claim 10 wherein the filter (80) is positioned downstream of the one-way expiratory valve (64).

## Patentansprüche

1. Maskenvorrichtung zur Zuführung eines Gases zur Inhalation mit:
einem Gesichtsteil (20);
einem Gasbehälter (40) zum Aufnehmen des Gases;
einem an dem Gesichtsteil (20) und dem Behälter (40) angeordneten Gehäuse (30), wobei das Gehäuse umfasst:
eine Gaseinlassöffnung (52), die das Gas zu dem Gesichtsteil (20) und dem Gasbehälter (40) führt;
ein Einweg-Expirationsventil (64), das sich in der Richtung der Expirationsströmung zur Atmosphäre hin öffnet; und
ein Einweg-Inspirationsventil (54), das zwischen dem Gesichtsteil (20) und dem Gasbehälter (40) angeordnet ist;
**dadurch gekennzeichnet, dass** das Gehäuse (30) weiterhin umfasst:
ein Verdünnungsventil (72), das sich öffnet und ermöglicht, dass Raumluft in das Gehäuse (30) eintritt, wobei das Verdünnungsventil (72) einen Öffnungsdruck aufweist, der größer ist als der zum Leeren des Behälters (40) während der Inspiration benötigte Druck, und einen Widerstand, der gleich oder kleiner ist als 4 cmH₂O/l/s bei einer Strömungsrate von 60 Litern pro Minute.

2. Maskenvorrichtung nach Anspruch 1, wobei das Inspirationsventil (54) einen Widerstand aufweist, der gleich oder kleiner als 2 cmH₂O/l/s ist.

3. Maskenvorrichtung nach Anspruch 1, wobei das Expirationsventil (64) einen Widerstand aufweist, der gleich oder kleiner als 2 cmH₂O/l/s ist.

4. Maskenvorrichtung nach Anspruch 1, wobei das Inspirationsventil (54) einen Widerstand aufweist, der kleiner ist als der des Verdünnungsventils (72).

5. Maskenvorrichtung nach Anspruch 1, die weiterhin ein Anti-Erstickungsventil (56) aufweist.

6. Maskenvorrichtung nach Anspruch 1, die weiterhin ein an dem Gesichtsteil (20) befestigtes Band zum Befestigen der Maskenvorrichtung am Gesicht eines Subjekts umfasst.

7. Maskenvorrichtung nach Anspruch 1, wobei der Gasbehälter ein Beutel (40) ist.

8. Maskenvorrichtung nach Anspruch 1, wobei das Gehäuse (30) durchsichtig ist.

9. Maskenvorrichtung nach einem der vorhergehenden Ansprüche, wobei die Ventile jeweils einen Ventilsitz mit einer Durchgangsöffnung und eine an einem Schaft angeordnete Flügelklappe aufweisen, wobei der Schaft sich durch die Durchgangsöffnung erstreckt, um die Klappe mechanisch an dem Ventilsitz zu sichern.

10. Maskenvorrichtung nach einem der vorhergehenden Ansprüche, wobei das Gehäuse einen Filter (80) umfasst, der entlang der Expirationsströmung positioniert ist.

11. Maskenvorrichtung nach Anspruch 10, wobei der Filter (80) geeignet ist, einen infektiösen Erreger auszufiltern.

12. Maskenvorrichtung nach Anspruch 10, wobei der Filter (80) dazu geeignet ist, ein Anästhesiegas herauszufiltern.

13. Maskenvorrichtung nach Anspruch 10, wobei der Filter (80) dazu geeignet ist, Kohlenstoffmonoxid herauszufiltern.

14. Maskenvorrichtung nach Anspruch 10, wobei der Filter (80) stromabwärts des Einwege-Expirationsventils (64) positioniert ist.

## Revendications

1. Ensemble formant masque destiné à délivrer un gaz pour l'inhalation, l'ensemble formant masque comprenant :
une pièce faciale (20) ;
un réservoir de gaz (40) pour contenir le gaz ;
un boîtier (30) fixé sur la pièce faciale (20) et le réservoir (40), dans lequel le boîtier comprend :
un orifice d'entrée de gaz (52) qui alimente le gaz à la pièce faciale (20) et au réservoir de gaz (40) ;
une vanne d'expiration unidirectionnelle (64) qui s'ouvre dans la direction du flux expiratoire menant à l'atmosphère ; et
une vanne d'inspiration unidirectionnelle (54) positionnée entre la pièce faciale (20) et le réservoir de gaz (40) ;
**caractérisé en ce que** le boîtier (30) comprend en outre :
une vanne de dilution (72) qui s'ouvre et permet à l'air ambiant d'entrer dans le boîtier (30), ladite vanne de dilution (72) ayant une pression de début d'écoulement supérieure à la pression nécessaire pour vider le réservoir (40) pendant l'inspiration et une résistance égale ou inférieure à 4 cmH₂O/l/sec à un débit de 60 litres par minute.

2. Ensemble formant masque selon la revendication 1, dans lequel la vanne d'inspiration (54) a une résistance égale ou inférieure à 2 cmH₂O/l/sec.

3. Ensemble formant masque selon la revendication 1, dans lequel la vanne d'expiration (64) a une résistance égale ou inférieure à 2 cmH₂O/l/sec.

4. Ensemble formant masque selon la revendication 1, dans lequel la vanne d'inspiration (54) a une résistance inférieure à la vanne de dilution (72).

5. Ensemble formant masque selon la revendication 1 comprenant en outre une vanne anti-asphyxie (56).

6. Ensemble formant masque selon la revendication 1, comprenant en outre une sangle fixée sur la pièce faciale (20) pour fixer l'ensemble formant masque sur le visage d'un sujet.

7. Ensemble formant masque selon la revendication 1, dans lequel le réservoir de gaz est un sac (40).

8. Ensemble formant masque selon la revendication 1, dans lequel le boîtier (30) est transparent.

9. Ensemble formant masque selon l'une quelconque des revendications précédentes, dans lequel les vannes comprennent chacune un siège de vanne ayant un trou d'orifice, une feuille d'obturation fixée sur une tige, dans lequel la tige s'étend à travers le trou d'orifice pour fixer mécaniquement l'obturateur sur le siège de vanne.

10. Ensemble formant masque selon l'une quelconque des revendications précédentes, dans lequel le boîtier comprend un filtre (80) positionné le long du flux d'expiration.

11. Ensemble formant masque selon la revendication 10, dans lequel le filtre (80) est capable de filtrer un agent infectieux.

12. Ensemble formant masque selon la revendication 10, dans lequel le filtre (80) est capable de filtrer un gaz anesthésiant.

13. Ensemble formant masque selon la revendication 10, dans lequel le filtre (80) est capable de filtrer du monoxyde de carbone.

14. Ensemble formant masque selon la revendication 10, dans lequel le filtre (80) est positionné en aval de la vanne d'expiration unidirectionnelle (64).
